Europäisches Patentamt

European Patent Office

Office européen des brevets

(11) Publication number: **0 200 470**
A1

# (12) EUROPEAN PATENT APPLICATION

(21) Application number: **86303062.3**

(22) Date of filing: **23.04.86**

(51) Int. Cl.⁴: **A 61 M 16/00**

(30) Priority: **23.04.85 AU 261/85**

(43) Date of publication of application:
**05.11.86 Bulletin 86/45**

(84) Designated Contracting States:
**DE FR GB**

(71) Applicant: **Waller, Raymond Frank**
**Staff Quarters Plenty Hospital**
**Rosanna Victoria(AU)**

(72) Inventor: **Waller, Raymond Frank**
**Staff Quarters Plenty Hospital**
**Rosanna Victoria(AU)**

(74) Representative: **Frost, Dennis Thomas et al,**
**WITHERS & ROGERS 4 Dyer's Buildings Holborn**
**London, EC1N 2JT(GB)**

(54) Support for a tracheotomy tube.

(57) A web or band (10) of flexible material for a tracheotomy tube (40) or an endotracheotomy tube (32), the material being adapted to be connected to the tube and to extend around the patient's head, the band having adjacent one end, an apertured portion (14) through which aperture the free end (11) of the band can pass, and associated or integral with the band a clip (21) comprising two members adapted to pass over and receive at least one thickness of the band, whereby the band can be retained in position.

FIG. 1.

FIG. 2.

EP 0 200 470 A1

This invention relates to improvements in tracheotomy and endotracheotomy tubes.

Where persons have had a tracheotomy, they are usually provided with a tube which can be considered to have a first member which is adapted to pass through the hole surgically formed in the trachea and to provide an airway therethrough, an enlarged surround, which is adapted to abut the surface of the patient's throat and to restrict the inward movement of the first member and, on occasions, a member extending outwardly from the surround which is in direct contact with the first member to form the complete airway.

Usually the surround has means whereby a cotton tape or the like can be connected thereto, which tape can be passed around the neck of the patient and acts to restrain the tracheotomy tube in the required position on and in the patient's throat.

I have found in practice that such arrangements, whilst being reasonably satisfactory in maintaining the tracheotomy tube in position, are not satisfactory in that the cotton tape needs to be replaced regularly and is not fully satisfactory during use as it can provide a surface to receive germs and it is not aesthetically satisfactory.

In another case, where a person is having an operation or the like, an endotracheotomy tube is used, which tube is passed through the patient's mouth or nose into the throat, and which tube is used to provide air and anesthetic and, on occasions, other gasses to the patent's lungs.

In these cases, it is normally desirable to hold the tube in position, but it will be appreciated that, depending upon the physical size of the patient, so the tube's position at the mouth could vary over a substantial range.

For that reason, in this case, it is desirable to provide a means whereby the tube can be correctly held in position, which means is connected to the endotracheotomy tube and passes about the patient's head.

An object of the invention is to provide in or associated with a tracheotomy or endotracheotomy tube a preferred method of retaining each such tube in its operative position.

The invention, in the broad sense, provides, for a tracheotomy tube, a web or band of flexible material which is adapted to be connected to an aperture in one side of the elongated surround on the tube, to pass around the neck of the user and to be connected to an aperture on the other side of the enlarged surround, characterised in that the web or band has, at one end, an apertured portion through which aperture the free end of the band can pass, and clamp means adapted to clamp overlying portions of the band, whereby connection to the said one side may be effected by passing the free end of the band through the aperture in the surround and through the aperture in the band and the connection at the other side may be effected by passing the free end of the band through the aperture in the surround, lying the portion of the band which passes through the aperture over the portion adjacent the aperture and retaining the two portions in this position by the clamp.

Preferably the band has the clamp member integrally formed adjacent its apertured end, which clamp member comprises two parts connected along a fold line and which are formed to receive and retain the two layers of the band, when the two parts are folded along their fold line, and retaining means whereby the two parts can be retained in the folded condition, which clamp member is adapted to be removed from the band before use.

In a second form of the invention, the band may be useful for retaining an endotracheotomy tube which does not have apertures to which a retaining band may be connected, a web or band of flexible material adapted to be connected to the tube and to retain the tube relative to a patient's head, the band having, adjacent one end, a first apertured portion through which the free end of the band can pass, a second apertured portion in connection with the first apertured portion and through which the free end of the band can pass and an integral clamp means extending from the second apertured portion whereby connection to the tube can be achieved by passing the free end of the band through the first apertured portion and placing the loop so formed over the tube, passing the band around the patient's head or neck, passing the free end of the band through the second apertured portion and retaining the end of the band which extends through the apertured portion by the clamp member.

The clamp member can be effectively identical to the clamp member described hereinabove and comprise two parts connected along a fold line.

It can be seen that the arrangement of the second form of the invention can also be used for the first form of the invention by cutting the band between the first and second apertured portions or between the second apertured portion and the clamp, and, if desired, at both of these positions, to provide a tube and a clamp which meet the requirements of the band for the tracheotomy tube.

In order that this invention can be more readily understood, I shall describe, in relation to the accompanying drawings, a particular form of band for a tracheotomy or an endotracheotomy tube made in accordance with the invention.

In these drawings:-

    Fig. 1    shows the band fitted to an endotracheotomy tube, the arrangement being somewhat stylized;

    Fig. 2    shows the band fitted to a tracheotomy tube;  and

    Fig. 3    is a perspective view of one end of the band.

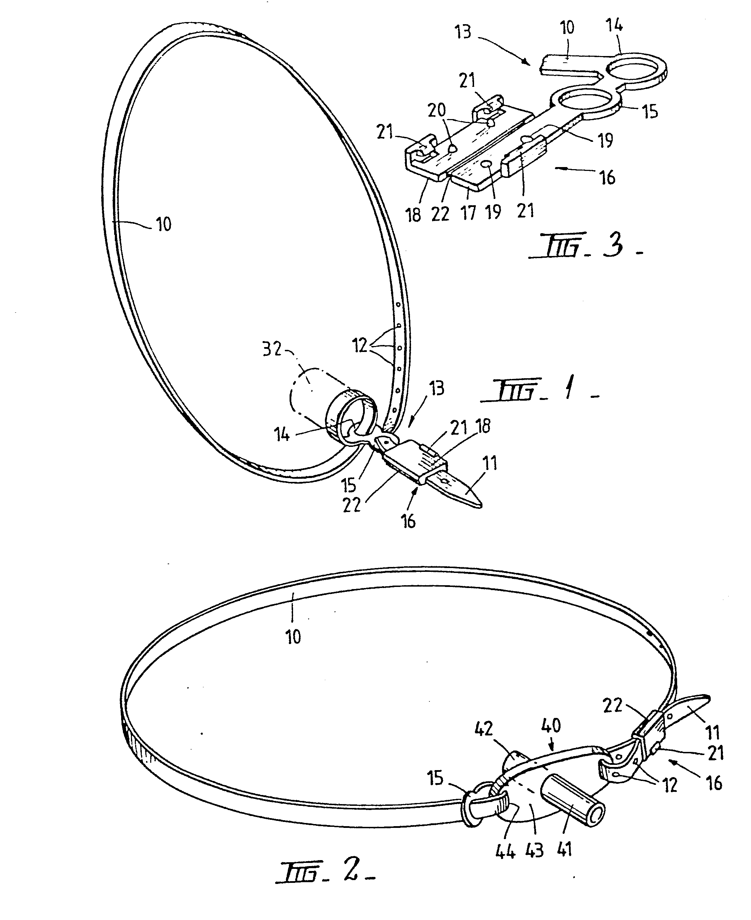

Referring, firstly, to Figs. 1 and 3, the actual construction of the band can be visualised.

The band could be formed from a sheet synthetic plastics material but, in order to form the clamp most satisfactorily, I prefer to injection mould the band.

The band itself comprises a body portion 10 which constitutes the major part of the total length of the band and is a length such as to be passed around the neck or head of the patient with whom it is to be used.

One end 11 is preferably formed to come to a relatively sharp end, to permit the band to be passed through receiving apertures, as will be described and, adjacent the end, there are a number of apertures 12, the purpose of which will, again, be later described, but which act in association with the clamp.   The number of these apertures 12 are such as to permit the band to be used with patients of various sizes.

The other end 13 of the band is shown in Fig. 3.

In this Figure, it can be seen that, formed integrally with the end of the portion 10 of the band, there is a first loop 14, the diameter of the apertures in which is such as to be able to be receive the band when the end 11 is passed therethrough. If required the actual diameter of the apertures in loop 14 may be slightly less than the width of the band so that this is somewhat deformed on passing through.

Formed integrally with the loop 14 and extending at an angle to the band 10, there is a second loop 15 and, formed integrally with this, is a clamp 16.

The clamp 16 can be considered to have two body portions 17 and 18, the portion 17 having a pair of apertures 19 which have a spacing the same as the spacing of the apertures 12 on the band and the side 18 has a pair of pegs 20 which are spaced at the same spacing as the apertures 19 and are adapted to enter these apertures.

Clip means 21 are provided whereby the body portions can be located into a face to face abutment when they are moved about the hinge portion 22.

I shall refer now to the arrangement of Fig. 1, which is the arrangement of an endotracheotomy tube.

The endotracheotomy tube 32 is received in a patient's mouth or nose whilst an anesthetic or other gas is applied. The tube 32 is normally a straightish tube which may have means whereby a tape can be retained thereon.

In this case, the tube must be held within the patient's mouth or nose and, in order to make such an arrangement, I pass the free end 11 of the band through the loop 14 and this forms a slip arrangement which can be located over the barrel 32 of the tube 30.

The band 10 is then passed around the back of the patient's head and the free end 11 is passed through the loop 15 and overlies body portion 17 of the clamp and the apertures 12 can be located in alignment with the apertures 19 on the body portion 17 and, then by rotation of the body portion 18 about hinge 22, the pins 20 pass through the apertures 12 and into the apertures 19 and the clips 21 locate the members 17 and 18 in abutment. It will be seen that the band then cannot move until the clips are released.

It will be seen that the arrangement of the invention provides a means whereby an endotracheotomy tube can readily be located in a patient's mouth and retained therein and the location and maintenance is basically very simple.

When the tube is to be released, it is only necessary to release the clips 21 so that the member 18 can move away from the member 17 freeing the end of the band and permitting it to pass through the loop 15, at which time the tube can be removed. Alternatively, the band can be cut

Exactly the same band can be used to maintain a normal tracheotomy tube in position in a patient's throat.

This arrangement is shown in Fig. 2, with the tracheotomy tube being shown as 40 and the tube has a forwardly extending member 41 through which air may pass and a rearwardly extending member 42, shown in broken line, which is received in the hole in the patient's trachea.

In this case, a band identical to the band previously described is used, but the clamp 16 is removed from the end, normally by cutting between the loops 14 and 15 or, if required, between the loop 15 and the clamp itself. In fact, the loop 15 is redundant to this application and can be removed completely.

Tracheotomy tubes have a base member 43, which is adapted to abut the patient's throat and this member has apertures 44 on each side.

In order to locate and retain the tracheotomy tube in the required position, the end 11 of the band is passed through one aperture 44 and through the loop 14 so that the loop assumes a position effectively as shown in Fig. 2, the portion 10 of the band is caused to pass around the patient's neck and the free end 11 is passed through the aperture 44 on the other side base member 43 and is turned back upon itself.

In this position, apertures 12 in the free end 11 overlie apertures 12 in the portion before connection to the tube and the clamp 16 is passed on each side of the band portions by rotation of the body portions 17, 18 about the hinge portion 22 and the clips 21 are caused to hold the portions 17, 18 in abutment with the pegs 20 passing through the apertures in the two portions of the band, thus firmly locating the tracheotomy tube.

In order to release the tracheotomy tube, it is only necessary to either unclip the clips on the clamp 16 or, simply, cut the band, at which time the tube can be removed.

It will be seen that, in each case, the arrangement is very satisfactory in practice, as the associated tube is firmly held, the band cannot slip or become released once it is properly located and, generally, the appearance of the device is more aesthetic than has previously been the case and, further, the device can be gas or radiation sterilised or autoclaved if this is required.

CLAIMS:

1. For a tracheotomy tube (40), a web or band (10) of flexible material which is adapted to be connected to an aperture (44) in one side of the elongated surround (43) on the tube (41), to pass around the neck of the user and to be connected to an aperture (44) on the other side of the enlarged surround (43), characterised in that the web or band has, at one end, an apertured portion (14) through which aperture the free end (11) of the band can pass, and clamp means (16) adapted to clamp overlying portions of the band, whereby connection to the said one side may be effected by passing the free end (11) of the band through the aperture (44) in the surround and through the aperture (14) in the band and the connection at the other side may be effected by passing the free end (11) of the band through the aperture (44) in the surround, lying the portion of the band which passes through the aperture over the portion adjacent the aperture and retaining the two portions in this position by the clamp (16)

2. A band as claimed in claim 1 having a clamp member integrally formed adjacent its apertured end.

3. For an endotracheotomy tube 32 which does not have apertures to which a retaining band may be connected, a web or band (10) of flexible material adapted to be connected to the tube and to retain the tube relative to a patient's head, the band having, adjacent one end, a first apertured portion (14) through which the free end (11) of the band can pass, a second apertured portion (15) in connection with the first apertured portion (14) and through which the free end of the band can pass and an integral clamp means (16) extending from the second apertured portion (15) whereby connection to the tube can be achieved by passing the free end (11) of the band through the first apertured portion (14) and placing the loop so formed over the tube (32), passing the band (10) around the patient's head or neck, passing the free end (11) of the band through the second apertured portion (15) and retaining the end of the band which extends through the apertured portion by the clamp member (16).

4. A band as claimed in any one of claims 1 to 3 wherein the clamp member comprises two parts (17,18) connected along a fold line (22) and which are formed to receive and retain the two layers of the band, when the two parts are folded along the fold line (22), and retaining means (21) whereby the two parts can be retained in the folded condition, which clamp member is adapted to be removed from the band before use.

5.   A band as claimed in claim 4 wherein the band is formed with a plurality of apertures (12) along its length and wherein the clamp member has means (19,20) to co-operate with such holes to locate the band in position.

6.   A band as claimed in claim 5 wherein the clamp member has on one part at least one stud (20) adapted to pass through a hole(s) (12) in the band and on the other part an aperture (19) corresponding to each stud (20) to receive the free end of the stud when located.

7.   A band as claimed in any one of claims 4 to 6 wherein the clamp means comprises a clip (21), components of which are on the outer sides of each part and which interengage when the parts are folded.

8.   A band as claimed in any previous claim which is made of a synthetic plastics material.

9.   The combination of a tracheotomy tube (40) and a band (10) as claimed in either claim 1 or claim 2 or any one of claims 4 to 8 when appended to either claim 1 or claim 2.

10.  A combination of an endotracheotomy tube (32) and a band (10) as claimed in claim 3 or in any one of claims 4 to 8 when appended to claim 3.

0200470

1/1

**FIG. 3.**

**FIG. 1.**

**FIG. 2.**

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | US-A-3 760 811 (ANDREW)<br>* Abstract; column 2, lines 7-9; column 4, lines 3-10; figure 6 *<br>--- | 1,4-7 | A 61 M 16/00 |
| Y | US-A-3 823 443 (KOHSHOH LIMITED)<br>* Abstract; column 4, lines 20-45; figure 10 *<br>--- | 1,4-7 | |
| A | US-A-2 765 792 (NICHOLS)<br>* Column 4, lines 11-15; figure 4 *<br>--- | 1 | |
| A | US-A-4 437 463 (ACKRAD LABORATORIES)<br>* Column 1, lines 37-47; figure 1 *<br>----- | 3,8 | TECHNICAL FIELDS SEARCHED (Int Cl 4) |
| | | | A 61 M<br>A 44 B<br>A 61 B |

The present search report has been drawn up for all claims

| Place of search | Date of completion of the search | Examiner |
|---|---|---|
| THE HAGUE | 22-07-1986 | SCHOENLEBEN J.E.F. |